# EUROPEAN PATENT APPLICATION

(11) **EP 1 806 410 A2**
(43) Date of publication of application: **11.07.2007**
(21) Application number: 06125593.1
(22) Date of filing: 19.08.2002
(51) Int. Cl.: C12P 19/34, C12Q 1/68, C07H 21/04, C07H 21/02

(54) **Fluorescent multiplex HPV PCR assays using multiple fluorophores**

(30) Priority: 23.08.2001 US 314383 P
(62) Divisional of application: 02759447.2
(71) Applicant: Merck & Co., Inc., Rahway, New Jersey 07065-0907 (US)
(72) Inventor: JANSEN, Kathrin, U., Rahway, NJ 07065-0907 (US); TADDEO, Frank, J., Rahway, NJ 07065-0907 (US); LI, Weili, Rahway, NJ 07065-0907 (US); DICELLO, Anthony, C., Rahway, NJ 07065-0907 (US)
(74) Representative: Buchan, Gavin MacNicol

(57) **Abstract**

The present invention relates a fluorescent multiplex PCR assay for detecting the presence of an HPV subtype in a sample using multiple fluorophores to simultaneously detect a plurality of HPV genes of the same HPV subtype. The present invention also relates to primer pairs and probes specific to HPV subtypes for use in the methods of the present invention.

SEQUENCE LISTING

<110> Merck & Co., Inc.
      Jansen, Kathrin
      Taddeo, Frank J.
      Li, Weili
      DiCello, Anthony C.

<120> FLUORESCENT MULTIPLEX HPV PCR ASSAYS
  USING MULTIPLE FLUOROPHORES

<130> 20847-PCT

<150> US 60/314,383
<151> 2001-08-23

<160> 36

<170> FastSEQ for Windows Version 4.0

<210> 1
<211> 24
<212> DNA
<213> Artificial Sequence

<220>
<223> HPV6 L1 PCR primer

<400> 1
gactcgtctc tttttgatcc caca                                              24

<210> 2
<211> 25
<212> DNA
<213> Artificial Sequence

<220>
<223> HPV6 L1 PCR primer

<400> 2
taggaaagga tgtccactta caccc                                             25

<210> 3
<211> 30
<212> DNA
<213> Artificial Sequence

<220>
<223> HPV6 L1 Probe

<400> 3
caacgtttgg tatgggcatg cacaggccta                                        30

<210> 4
<211> 21
<212> DNA
<213> Artificial Sequence

<220>
<223> HPV6 E6 PCR primer

<400> 4
taaaggtcct gtttcgaggc g                                                21

<210> 5
<211> 23
<212> DNA
<213> Artificial Sequence

<220>
<223> HPV6 E6 PCR primer

<400> 5
tgacacaggt agcaccgaat tag                                              23

<210> 6
<211> 27
<212> DNA
<213> Artificial Sequence

<220>
<223> HPV6 E6 Probe

<400> 6
atccatatgc agcctgcgcg tgctgcc                                          27

<210> 7
<211> 22
<212> DNA
<213> Artificial Sequence

<220>
<223> HPV6 E7 PCR primer

<400> 7
acgaagtgga cggacaagat tc                                               22

<210> 8
<211> 22
<212> DNA
<213> Artificial Sequence

<220>
<223> HPV6 E7 PCR primer

<400> 8
tcccaacaga agctgttgca ct                                               22

<210> 9
<211> 30
<212> DNA
<213> Artificial Sequence

<220>
<223> HPV6E7 Probe

<400> 9
ctgtacactg cacaaccagt cgaacgttgc                                        30

<210> 10
<211> 24
<212> DNA
<213> Artificial Sequence

<220>
<223> HPV11L1 PCR primer

<400> 10
cctccaccaa atggtacact ggag                                              24

<210> 11
<211> 24
<212> DNA
<213> Artificial Sequence

<220>
<223> HPV11L1 PCR primer

<400> 11
cctccaccaa atggtacact ggag                                              24

<210> 12
<211> 29
<212> DNA
<213> Artificial Sequence

<220>
<223> HPV11L1 Probe

<400> 12
cagtcacagg ccattacctg tcagaaacc                                         29

<210> 13
<211> 25
<212> DNA
<213> Artificial Sequence

<220>
<223> HPV11E6 PCR primer

<400> 13
tttgcacact ctgcaaattc agtgc                                             25

<210> 14
<211> 26
<212> DNA
<213> Artificial Sequence

<220>
<223> HPV11E6 PCR primer

<400> 14
ttgcagttct aagcaacagg cacacg                                            26

<210> 15
<211> 30
<212> DNA
<213> Artificial Sequence

<220>
<223> HPV11E6 Probe

<400> 15
catatatctc tgcggtggtc agtgcattcc                                        30

<210> 16
<211> 24
<212> DNA
<213> Artificial Sequence

<220>
<223> HPV11E7 PCR primer

<400> 16
agctcagaag atgaggtgga caag                                              24

<210> 17
<211> 22
<212> DNA
<213> HPV11E7 PCR prArtificial Sequence

<220>
<223> HPV11E7 PCR primer

<400> 17
tgcccagcaa aaggtcttgt ag                                                22

<210> 18
<211> 26
<212> DNA
<213> Artificial Sequence

<220>
<223> HPV11E7 Probe

<400> 18
cactccacaa ccagtcggac gttgct                                            26

<210> 19
<211> 20
<212> DNA
<213> Artificial Sequence

<220>
<223> HPV16L1 PCR primer

<400> 19
tccagataca cagcggctgg                                                    20

<210> 20
<211> 24
<212> DNA
<213> Artificial Sequence

<220>
<223> HPV16L1 PCR primer

<400> 20
aataaaggat ggccactaat gccc                                               24

<210> 21
<211> 27
<212> DNA
<213> Artificial Sequence

<220>
<223> HPV16L1 Probe

<400> 21
aatggctgac cacgacctac ctcaaca                                            27

<210> 22
<211> 22
<212> DNA
<213> Artificial Sequence

<220>
<223> HPV 16E6 PCR primer

<400> 22
gcgacccaga aagttaccac ag                                                 22

<210> 23
<211> 29
<212> DNA
<213> Artificial Sequence

<220>
<223> HPV 16E6 PCR primer

<400> 23
ccatctctat atactatgca taaatcccg                                          29

<210> 24
<211> 28
<212> DNA
<213> Artificial Sequence

<220>
<223> HPV 16E6 Probe

<400> 24
tacctcacgt cgcagtaact gttgcttg                                          28

<210> 25
<211> 22
<212> DNA
<213> Artificial Sequence

<220>
<223> HPV 16E7 PCR primer

<400> 25
agaaccggac agagcccatt ac                                                22

<210> 26
<211> 23
<212> DNA
<213> Artificial Sequence

<220>
<223> HPV 16E7 PCR primer

<400> 26
gcccattaac aggtcttcca aag                                               23

<210> 27
<211> 28
<212> DNA
<213> Artificial Sequence

<220>
<223> HPV 16E7 Probe

<400> 27
cgcacaaccg aagcgtagag tcacactt                                          28

<210> 28
<211> 19
<212> DNA
<213> Artificial Sequence

<220>
<223> HPV18L1 PCR primer

<400> 28
ttggttcagg ctggattgc                                                    19

<210> 29
<211> 20
<212> DNA
<213> Artificial Sequence

<220>
<223> HPV18L1 PCR primer

<400> 29
gcttggcagg tttagaagac                                                    20

<210> 30
<211> 29
<212> DNA
<213> Artificial Sequence

<220>
<223> HPV18L1 Probe

<400> 30
cctcgcaaac gttctgctcc atctgccac                                          29

<210> 31
<211> 19
<212> DNA
<213> Artificial Sequence

<220>
<223> HPV18E6 PCR primer

<400> 31
agctgggcac tatagaggc                                                     19

<210> 32
<211> 21
<212> DNA
<213> Artificial Sequence

<220>
<223> HPV18E6 PCR primer

<400> 32
tgtgtttctc tgcgtcgttg g                                                  21

<210> 33
<211> 26
<212> DNA
<213> Artificial Sequence

<220>
<223> HPV18E6 Probe

<400> 33
ccattcgtgc tgcaaccgag cacgac                                             26

<210> 34
<211> 21
<212> DNA
<213> Artificial Sequence

<220>
<223> HPV18E7 PCR primer

<400> 34
gaaccacaac gtcacacaat g                                                21

<210> 35
<211> 20
<212> DNA
<213> Artificial Sequence

<220>
<223> HPV18E7 PCR primer

<400> 35
cagaaacagc tgctggaatg                                                  20

<210> 36
<211> 33
<212> DNA
<213> Artificial Sequence

<220>
<223> HPV18E7 Probe

<400> 36
tctgctgagc tttctactac tagctcaatt ctg                                   33

## Description

### FIELD OF THE INVENTION

The present invention relates generally to PCR-based assays to detect the presence of human papillomavirus (HPV) subtypes in clinical samples. More specifically, it relates to a fluorescent multiplex PCR assay, wherein multiple fluorophores are used to simultaneously detect a plurality of HPV loci in a single PCR reaction tube.

### BACKGROUND OF THE INVENTION

There are more than 80 types of human papillomavirus (HPV) that cause a wide variety of biological phenotypes, from benign proliferative warts to malignant carcinomas (for review, see McMurray et al., Int. J. Exp. Pathol. 82(1): 15-33 (2001)). HPV6 and HPV11 are the types most commonly associated with benign warts, whereas HPV16 and HPV18 are the high-risk types most frequently associated with malignant lesions. Determination of the specific type of HPV in a clinical sample is, therefore, critical for predicting risk of developing HPV-associated disease.

Several nucleic acid-based methods have been utilized to identify and quantify specific HPV types in clinical samples, such as detection of viral nucleic acid by in situ hybridization, Southern blot analysis, hybrid capture or polymerase chain reaction (PCR). PCR-based methods often involve amplification of a single specific HPV target sequence followed by blotting the resulting amplicon to a membrane and probing with a radioactively labeled oligonucleotide probe.

Other methods exploit the high homology between specific HPV genes of different subtypes through the use of commercially available consensus primers capable of PCR amplifying numerous HPV subtypes present in a sample. The presence of a specific HPV subtype is then identified using a subtype-specific oligonucleotide probe. See, e.g., Kleter et al., Journal of Clinical Microbiology 37(8): 2508-2517 (1999); Gravitt et al., Journal of Clinical Microbiology 38(1): 357-361 (2000). Similarly, assays that utilize degenerate PCR primers take advantage of the homology between HPV subtypes, allowing detection of a greater number of HPV types than methods utilizing specific primer sets. See, e.g. Harwood et al., Journal of Clinical Microbiology 37(11): 3545-3555 (1999). Such assays also require additional experimentation to identify specific HPV subtypes.

The PCR methods described above can be associated with several problems. For example, differences in reaction efficiencies among HPV subtypes can result in disproportionate amplification of some subtypes relative to others. Additionally, the equilibrium for amplification will be driven towards those subtypes that exist at higher copy numbers in a sample, which will consume the PCR reaction components, thus making amplification of the minor HPV subtypes less likely.

Also described in the art is a 5' exonuclease fluorogenic PCR-based assay (Taq-Man PCR) which allows detection of PCR products in real-time and eliminates the need for radioactivity. See, e.g., U.S. Patent No. 5,538,848; Holland et al, Proc. Natl. Acad. Sci. USA 88: 7276-7280 (1991). This method utilizes a labeled probe, comprising a fluorescent reporter (fluorophore) and a quencher, that hybridizes to the target DNA between the PCR primers. Excitation of the fluorophore results in the release of a fluorescent signal by the fluorophore which is quenched by the quencher. Amplicons can be detected by the 5' - 3' exonuclease activity of the TAQ DNA polymerase, which degrades double-stranded DNA encountered during extension of the PCR primer, thus releasing the fluorophore from the probe. Thereafter, the fluorescent signal is no longer quenched and accumulation of the fluorescent signal, which is directly correlated with the amount of target DNA, can be detected in real-time with an automated fluorometer.

Taq-Man PCR assays have been adapted for HPV subtype detection. Swan et al. (Journal of Clinical Microbiology 35(4): 886-891 (1997)) disclose a fluorogenic probe assay that utilizes type-specific HPV primers that amplify a portion of the L1 gene in conjunction with type-specific probes. The Swan et al. assay measures fluorescent signal at the end of a fixed number of PCR cycles (endpoint reading) and not in real-time.

Josefsson et al. (Journal of Clinical Microbiology 37(3): 490-96 (1999)) report a Taq-Man assay that targets a highly conserved portion of the E1 gene in conjunction with type-specific probes labeled with different fluorescent dyes. A number of HPV types were amplified by utilizing a mixture of specific and degenerate primers. Josefsson et al. utilized up to three type-specific probes per assay, which were designed to detect a portion of the E1 gene from different HPV subtypes. Unlike the Swan et al. assay, Josefsson et al. measured the accumulation of fluorescence in real-time.

Tucker et al. (Molecular Diagnosis 6(1): 39-47 (2001)) describe an assay that targets a conserved region spanning the E6/E7 junction. Like the Josefsson assay, Tucker et al. employed real-time detection and type-specific fluorescent probes. Tucker et al. also utilized multiplex PCR to simultaneously detect HPV target sequences and either the actin or globin cellular loci in the same reaction tube.

The methods described above typically involve testing for the presence of a single viral locus in a DNA sample such as the L1 locus. A disadvantage of single-locus assays is that the high degree of homology among specific HPV genes from one HPV type to another leads to an excessive occurrence of false positive results. This level of homology makes it difficult to design a PCR assay that is specific for a single HPV type. It is therefore necessary to confirm positive results by testing for the presence of several loci of a single HPV-type. The further experimentation required to verify positive results is cumbersome and time-consuming. Establishment of the HPV status of a clinical sample for four different HPV types typically consumes 26-30 man-hours.

Single-locus assays may also lead to false negative results. It is well established that the relationship between the HPV genome and chromosomal host DNA may change during the multistage tumorigenic process (For review, see McMurray et al., Int. J. Exp. Path. 82: 15-33 (2001)). Premalignant lesions are often associated with episomal forms of HPV DNA while later-stage tumors typically have integrated HPV sequences. As a result of the integration correlated with advanced stages of disease progression, the open reading frame of specific HPV genes, such as the L1 gene, may become disrupted. Such disruption of HPV gene sequences may lead to false negative results in assays that target the disrupted sequence.

Despite the development of the HPV assays described above, it would be advantageous to develop an assay that is highly sensitive and reproducible, and that requires reduced man-hours compared to methods disclosed in the art.

### SUMMARY OF THE INVENTION

The present invention relates to a fluorescent multiplex PCR assay for detecting the presence of an HPV subtype in a sample which uses multiple fluorophores to simultaneously detect a plurality of HPV loci of the same HPV subtype.

More specifically, the present invention relates to a method for detecting the presence of a human papillomavirus (HPV) subtype in a nucleic acid-containing sample comprising:
amplifying the nucleic acid in the presence of a nucleic acid polymerase and a plurality of oligonucleotide sets to produce a plurality of PCR amplicons;
wherein each oligonucleotide set consists of (a) a forward discriminatory PCR primer hybridizing to a first location of an HPV subtype, (b) a reverse discriminatory PCR primer hybridizing to a second location of the HPV subtype downstream of the first location, and (c) a fluorescent probe labeled with a quencher molecule and a fluorophore which emits energy at a unique emission maxima; said probe hybridizing to a location of the HPV subtype between the first and the second locations;
wherein each oligonucleotide set specifically hybridizes to a different HPV amplicon derived from the same HPV subtype;
allowing said nucleic acid polymerase to digest each fluorescent probe during amplification to dissociate said fluorophore from said quencher molecule;
detecting a change of fluorescence upon dissociation of the fluorophore and the quencher, the change of fluorescence corresponding to the occurrence of nucleic acid amplification; and
determining that the sample is positive for the HPV subtype if a change of fluorescence is detected in at least two emission maxima.

In a preferred embodiment of this invention, each oligonucleotide set of the plurality of oligonucleotide sets is specific to a single gene of the HPV subtype to be detected. In other words, each oligonucleotide set of the method of the present invention hybridizes to nucleotide sequences derived from a single HPV gene of the same subtype. For example, the oligonucleotide primers and probe of a first oligonucleotide set hybridize to the E6 gene, the oligonucleotide primers and probe of a second oligonucleotide set hybridize to the E7 gene and the oligonucleotide primers and probe of a third oligonucleotide set hybridize to the L1 gene. As a result, a plurality of PCR amplicons is created wherein each PCR amplicon is specific to a single HPV gene of the HPV subtype to be detected.

In an alternative embodiment of this invention, the forward discriminatory PCR primer and the reverse discriminatory PCR primer of at least one oligonucleotide set are specific to a different gene of the same HPV subtype. For example, a forward discriminatory primer hybridizes to the E6 gene and a reverse discriminatory primer hybridizes to the E7 gene. As a result, at least one PCR amplicon comprises a sequence of nucleotides derived from more than one gene. The oligonucleotide probe specific to said amplicon may hybridize, for example, to a sequence of nucleotides derived from the E6 gene, a sequence of nucleotides derived from the E7 gene, or a sequence of nucleotides that crosses the E6/E7 boundary.

In a preferred embodiment of this invention, the HPV subtype is selected from the group consisting of: HPV6, HPV11, HPV 16 and HPV 18.

In a further preferred embodiment of the method of the present invention, the number of oligonucleotide sets is three and the oligonucleotide sets specifically hybridize to the E6, E7 and L1 genes of HPV. A sample is positive for the HPV subtype being tested if two or three of the E6, E7 or L1 genes are detected.

Another embodiment of this invention relates to an oligonucleotide probe comprising a sequence of nucleotides specific to a single HPV type. Said oligonucleotide probe can bind to specific HPV amplicons resulting from PCR amplification of viral DNA using specific oligonucleotide primers. In a further embodiment of this invention, said oligonucleotide probe comprises a sequence of nucleotides selected from the group consisting of: SEQ ID NO:3, SEQ ID NO:6, SEQ ID NO:9, SEQ ID NO:12, SEQ ID NO:12, SEQ ID NO:15, SEQ ID NO:18, SEQ ID NO:21, SEQ ID NO:24, SEQ ID NO:27, SEQ ID NO: 30, SEQ ID NO: 33 and SEQ ID NO:36.

The present invention also relates to said oligonucleotide probes further comprising a fluorophore and a quencher molecule. In a preferred embodiment of this invention, the fluorophore is selected from the group consisting of: FAM, JOE and TET and the quencher is non-fluorescent. In an especially preferred embodiment of this invention, the quencher is BHQ1.

The present invention further relates to a primer pair for the PCR amplification of HPV nucleic acid, wherein both the forward and reverse PCR primers are discriminatory. In a preferred embodiment of the invention, the nucleotide sequences of the primer pair are selected from the group consisting of: SEQ ID NO:1 and SEQ ID NO:2, SEQ ID NO:4 and SEQ ID NO:5, SEQ ID NO:7 and SEQ ID NO:8, SEQ ID NO:10 and SEQ ID NO:11, SEQ ID NO:13 and SEQ ID NO:14, SEQ ID NO:16 and SEQ ID NO:17, SEQ ID NO:19 and SEQ ID NO:20, SEQ ID NO:22 and SEQ ID NO:23, SEQ ID NO:25 and SEQ ID NO:26, SEQ ID NO:28 and SEQ ID NO:29, SEQ ID NO:31 and SEQ ID NO:32, and SEQ ID NO:34 and SEQ ID NO:35.

As used herein, the term "oligonucleotide" refers to linear oligomers of natural or modified monomers or linkages, including deoxyribonucleosides, ribonucleosides, and the like, capable of specifically binding to a target polynucleotide by way of a regular pattern of monomer-to-monomer interactions, such as Watson-Crick type base pairing. For purposes of this invention, the term oligonucleotide includes both oligonucleotide probes and oligonucleotide primers.

As used herein, the term "primer" refers to an oligonucleotide that is capable of acting as a point of initiation of synthesis along a complementary strand when placed under conditions in which synthesis of a primer extension product which is complementary to a nucleic acid strand is catalyzed. Such conditions include the presence of four different deoxyribonucleoside triphosphates and a polymerization-inducing agent such as DNA polymerase or reverse transcriptase, in a suitable buffer ("buffer" includes components which are cofactors, or which affect ionic strength, pH, etc.), and at a suitable temperature. As employed herein, an oligonucleotide primer can be naturally occurring, as in a purified restriction digest, or be produced synthetically. The primer is preferably single-stranded for maximum efficiency in amplification.

As used herein, "primer pair" refers to two primers, a forward primer and a reverse primer, that are capable of participating in PCR amplification of a segment of nucleic acid in the presence of a nucleic acid polymerase to produce a PCR amplicon. The primers that comprise a primer pair can be specific to the same HPV gene, resulting in an amplicon that consists of a sequence of nucleotides derived from a single HPV gene. Alternatively, the primers that comprise a primer pair can be specific to different HPV genes that reside within close proximity to each other within the HPV genome, thereby producing amplicons that consist of a sequence of nucleotides derived from more than one gene.

As used herein, "unique," in reference to the fluorophores of the present invention, means that each fluorophore emits energy at a differing emission maxima relative to all other fluorophores used in the particular assay. The use of fluorophores with unique emission maxima allows the simultaneous detection of the fluorescent energy emitted by each of the plurality of fluorophores used in the particular assay.

As used herein, the term "discriminatory," used in reference to the oligonucleotide primers and probes of the present invention, means that said primers and probes are specific to a single HPV subtype. It includes HPV primers and probes specific to a single HPV subtype, but that share some homology with other HPV subtypes. "Discriminatory" primers and probes of the present invention include those oligonucleotides that lack 3' homology with other HPV subtypes in at least one nucleotide or more. Such a residue that is unique for the specific HPV subtype at the specific position and acts to discriminate the HPV subtype from the others in the alignment referred to as a "discriminatory base". The term "discriminatory," in reference to oligonucleotides, does not include primers and probes that are specific to more than one HPV subtype, i.e. those that share full homology with greater than one HPV subtype.

As used herein, "amplicon" refers to a specific product of a PCR reaction, which is produced by PCR amplification of a sample comprising nucleic acid in the presence of a nucleic acid polymerase and a specific primer pair. An amplicon can consist of a nucleotide sequence derived from a single gene of a single HPV subtype or an amplicon can consist of a nucleotide sequence derived from more than one gene of a single HPV subtype.

As used herein, "oligonucleotide set" refers to a grouping of a pair of oligonucleotide primers and an oligonucleotide probe that hybridize to a specific nucleotide sequence of a single HPV subtype. Said oligonucleotide set consists of: (a) a forward discriminatory primer that hybridizes to a first location of an HPV subtype; (b) a reverse discriminatory primer that hybridizes to a second location of the HPV subtype downstream of the first location and (c) a fluorescent probe labeled with a fluorophore and a quencher, which hybridizes to a location of the HPV subtype between the primers. In other words, an oligonucleotide set consists of a set of specific PCR primers capable of initiating synthesis of an amplicon specific to a single HPV subtype, and a fluorescent probe which hybridizes to the amplicon.

As used herein, "plurality" means two or more.

As used herein, "specifically hybridizes," in reference to oligonucleotide sets, oligonucleotide primers or oligonucleotide probes, means that said oligonucleotide sets, primers or probes hybridize to a single HPV subtype.

As used herein, "gene" means a segment of nucleic acid involved in producing a polypeptide chain. It includes both translated sequences (coding region) and 5' and 3' untranslated sequences (non-coding regions) as well as intervening sequences (introns) between individual coding segments (exons). For purposes of this invention, the HPV genome has nine genes: L1, L2, and E1 - E7.

As used herein, "locus" refers to the position on a chromosome at which the gene for a trait resides. The term locus includes any one of the alleles of a specific gene. It also includes homologous genes from different HPV subtypes. For example, PCR assays that detect the L1 gene in HPV16 and HPV6 are single-locus assays, despite the detection of sequences from different HPV subtypes. Contrarily, for example, assays that detect the L1 gene and the E1 gene of a single HPV type are multiple locus assays, even though a single HPV subtype is detected.

As used herein, "HPV" means human papillomavirus. "HPV" is a general term used to refer to any subtype of HPV, whether currently known or subsequently described.

As used herein, "fluorophore" refers to a fluorescent reporter molecule which, upon excitation with a laser, tungsten, mercury or xenon lamp, or a light emitting diode, releases energy in the form of light with a defined spectrum. Through the process of fluorescence resonance energy transfer (FRET), the light emitted from the fluorophore can excite a second molecule whose excitation spectrum overlaps the emission spectrum of the fluorophore. The transfer of emission energy of the fluorophore to another molecule quenches the emission of the fluorophore. The second molecule is known as a quencher molecule. The term "fluorophore" is used interchangeably herein with the term "fluorescent reporter".

As used herein "quencher" or "quencher molecule" refers to a molecule that, when linked to a fluorescent probe comprising a fluorophore, is capable of accepting the energy emitted by the fluorophore, thereby quenching the emission of the fluorophore. A quencher can be fluorescent, which releases the accepted energy as light, or non-fluorescent, which releases the accepted energy as heat, and can be attached at any location along the length of the probe.

As used herein "dark quencher" refers to a non-fluorescent quencher.

As used herein, "probe" refers to an oligonucleotide that is capable of forming a duplex structure with a sequence in a target nucleic acid, due to complementarity of at least one sequence of the probe with a sequence in the target region, or region to be detected. The term "probe" includes an oligonucleotide as described above, with or without a fluorophore and a quencher molecule attached. The term "fluorescent probe" refers to a probe comprising a fluorophore and a quencher molecule.

As used herein, "FAM" refers to the fluorophore 6-carboxy-fluorescein.

As used herein "JOE" refers to the fluorophore 6-carboxy-4',5'-dichloro-2',7'-dimethoxyfluorescein.

As used herein, "TET" refers to the fluorophore 5-tetrachloro-fluorescein.

### BRIEF DESCRIPTION OF THE DRAWINGS

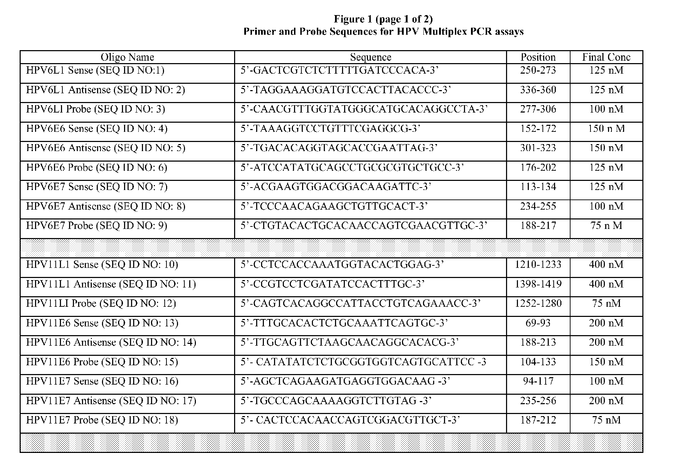

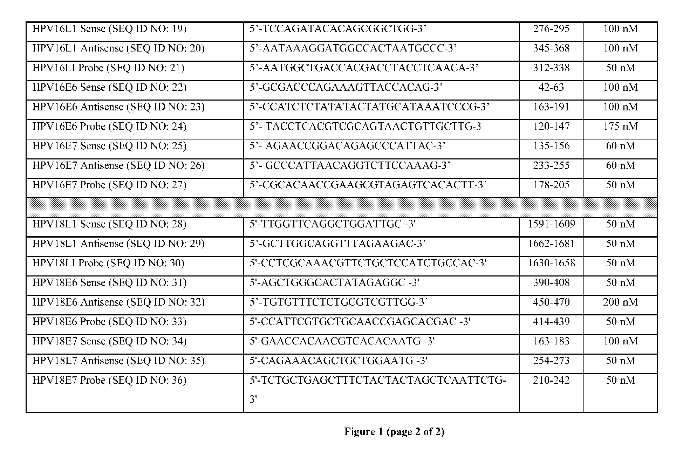

FIGURE 1 shows the sequence of the oligonucleotide primers and probes used in the TaqMan multiplex PCR reactions, their positions within the open reading frame (A of start codon = 1), and the final concentration of each oligo in the multiplex PCR reaction. The six primers for each HPV type were combined into one concentrated (100X) hexa-primer stock. Each probe was stored as a concentrated (100X) stock.

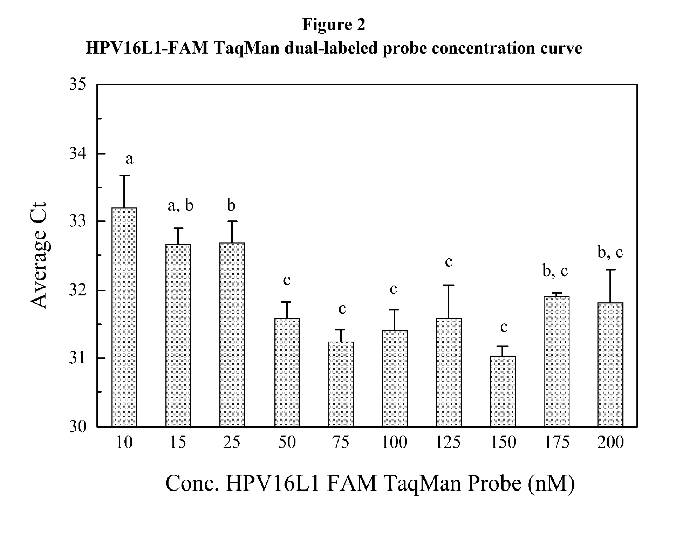

FIGURE 2 shows a HPV16L1-FAM TaqMan dual-labeled probe concentration curve. The mean (n=3) threshold cycle ± SD was determined for escalating HPV16L1 probe concentrations in a 50 µl TaqMan PCR reaction using 100 copies of HPV16L1 plasmid as template DNA. Columns with the same letter are not significantly different (p>0.05) based on one-way ANOVA and Tukey's multiple comparison post-test analyses.

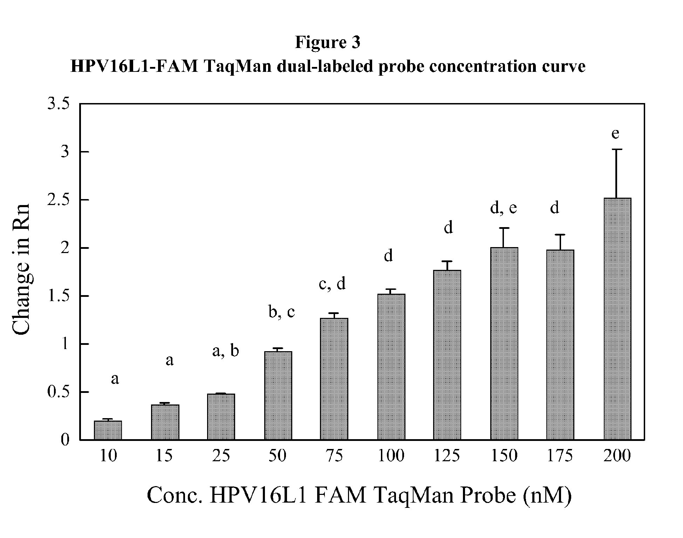

FIGURE 3 shows a HPV16L1-FAM TaqMan dual-labeled probe concentration curve. The mean (n=3) ΔRn (Rn-baseline) ± SD was determined for escalating HPV16L1 probe concentrations in a 50 µl TaqMan PCR reaction using 100 copies of HPV16L1 plasmid as template DNA. Columns with the same letter are not significantly different (p>0.05) based on one-way ANOVA and Tukey's multiple comparison post-test analyses.

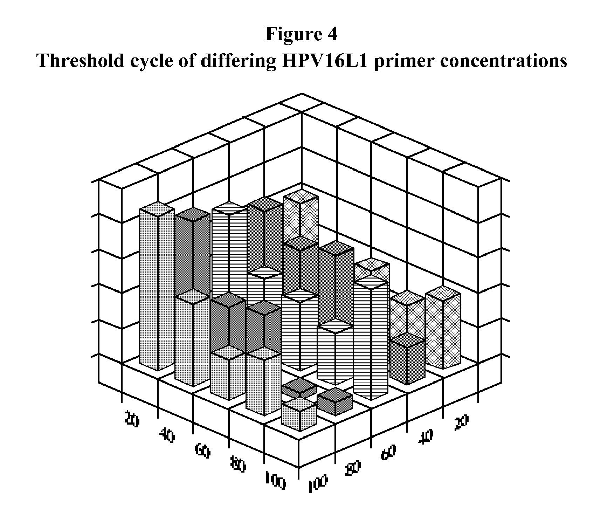

FIGURE 4 depicts the threshold cycle of differing HPV16L1 primer concentrations. Both the sense and antisense primers were tested in combination at differing concentrations using 10 copies of HPV16L1 plasmid as the DNA template.

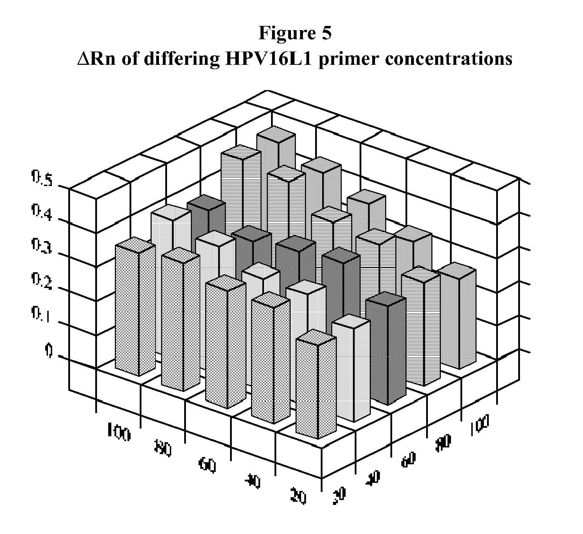

FIGURE 5 shows the ΔRn of differing HPV16L1 primer concentrations. Both the sense and antisense primers were tested in combination at differing concentrations using 10 copies of HPV16L1 plasmid as the DNA template.

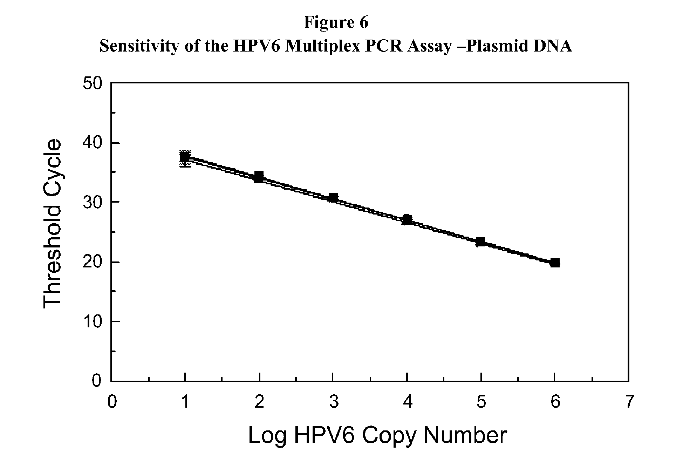

FIGURE 6 shows the sensitivity of the HPV6 multiplex PCR assay. Results (mean ± SD, n=3) obtained with each specific probe are depicted by different symbols: squares represent the HPV6L1-FAM probe, circles represent the HPV6E6-JOE probe and triangles represent the HPV6E7-TET probe.

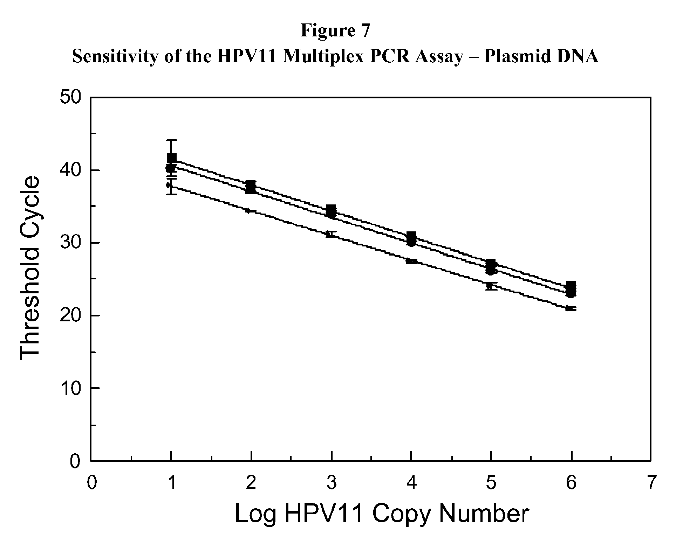

FIGURE 7 shows the sensitivity of the HPV11 multiplex PCR assay. Results (mean ± SD, n=3) obtained with each specific probe are depicted by different symbols: squares represent the HPV11L1-FAM probe; circles represent the HPV11E6-JOE probe and triangles represent the HPV11E7-TET probe.

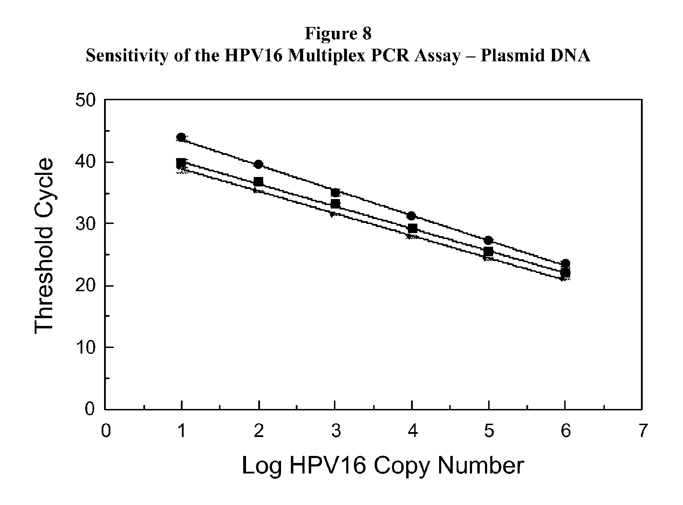

FIGURE 8 shows the sensitivity of the HPV16 multiplex PCR assay. Results (mean ± SD, n=3) obtained with each specific probe are depicted by different symbols: squares represent the HPV16L1-FAM probe; circles represent the HPV16E6-JOE probe and triangles represent the HPV16E7-TET probe.

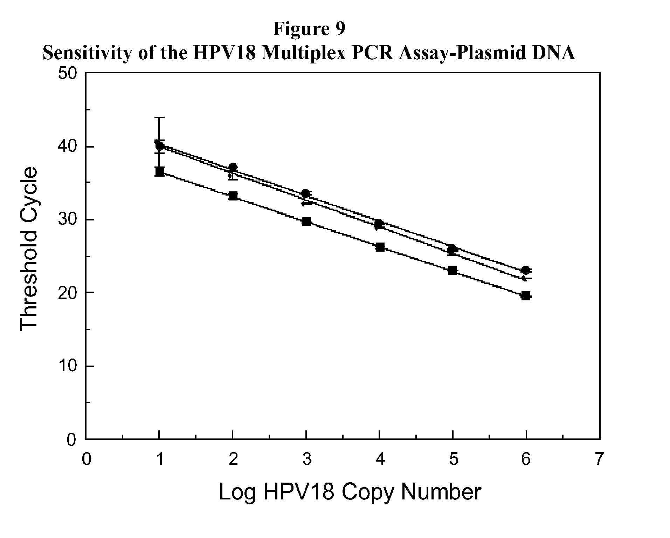

FIGURE 9 shows the sensitivity of the HPV18 multiplex PCR assay. Results (mean ± SD, n=3) obtained with each specific probe are depicted by different symbols: squares represent the HPV18L1-FAM probe; circles represent the HPV18E6-JOE probe and triangles represent the HPV18E7-TET probe.

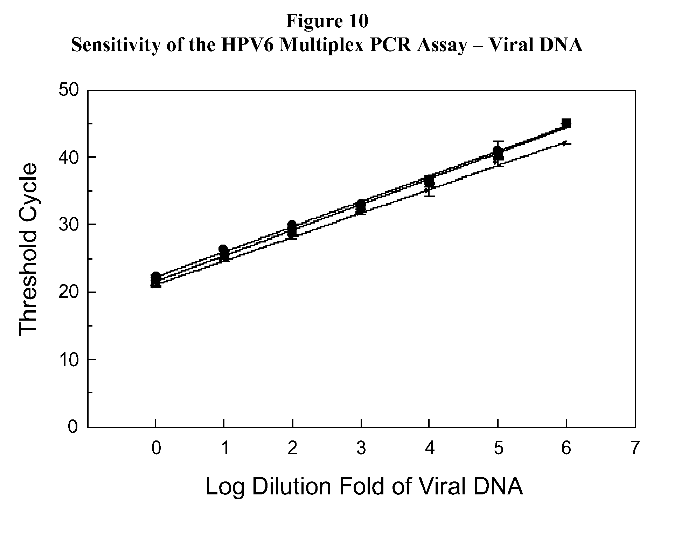

FIGURE 10 shows the sensitivity of the HPV6 multiplex PCR assay using a serial dilution of viral DNA purified from a human clinical specimen. Results (mean ± SD, n=3) obtained with each specific probe are depicted by different symbols: squares represent the HPV6L1-FAM probe; circles represent the HPV6E6-JOE probe and triangles represent the HPV6E7-TET probe.

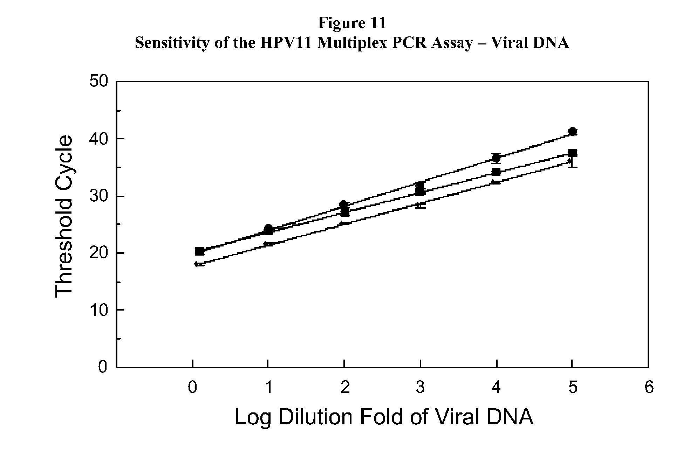

FIGURE 11 shows the sensitivity of the HPV11 multiplex PCR assay using a serial dilution of viral DNA purified from a human clinical specimen. Results (mean ± SD, n=3) obtained with each specific probe are depicted by different symbols: squares represent the HPV11L1-FAM probe; circles represent the HPV11E6-JOE probe and triangles represent the HPV11E7-TET probe.

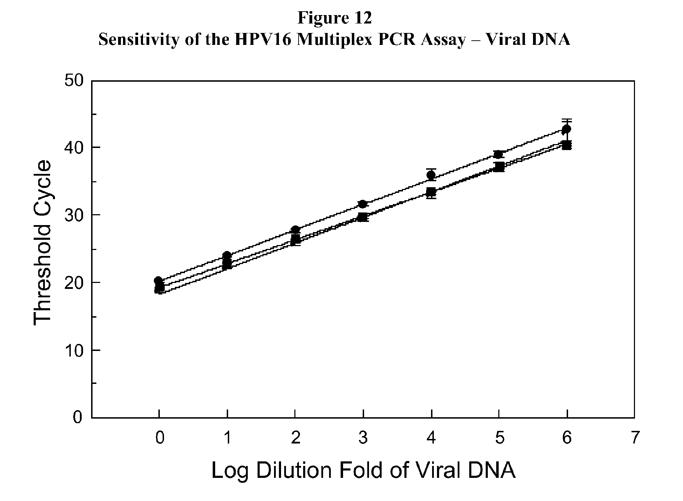

FIGURE 12 shows the sensitivity of the HPV16 multiplex PCR assay using a serial dilution of viral DNA purified from a human clinical specimen. Results (mean ± SD, n=3) obtained with each specific probe are depicted by different symbols: squares represent the HPV16L1-FAM probe; circles represent the HPV16E6-JOE probe and triangles represent the HPV16E7-TET probe.

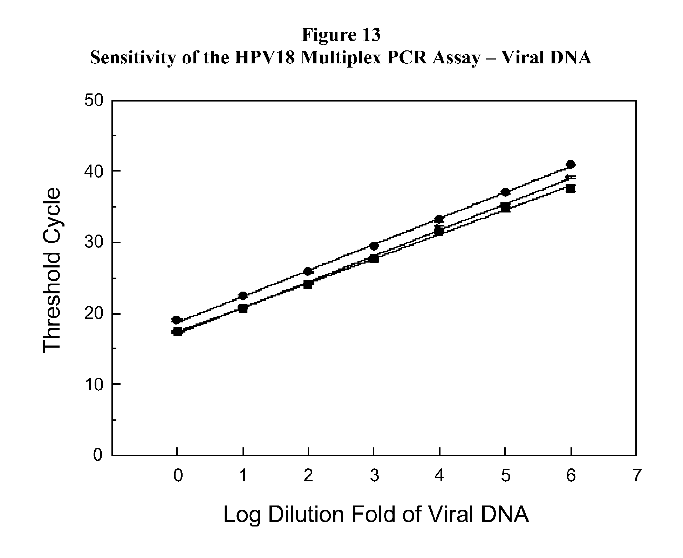

FIGURE 13 shows the sensitivity of the HPV18 multiplex PCR assay using a serial dilution of viral DNA purified from a Human clinical specimen. Results (mean ± SD, n=3) obtained with each specific probe are depicted by different symbols: squares represent the HPV18L1-FAM probe; circles represent the HPV18E6-JOE probe and triangles represent the HPV18E7-TET probe.

### DETAILED DESCRIPTION OF THE INVENTION

This invention relates to an assay for detection of HPV subtypes in a clinical sample that substantially reduces the risk of false negative results as compared to other assays known in the art.

It is well known that the relationship between the HPV genome and chromosomal host DNA may change during the multistage tumorigenic process (For review, see McMurray et al., Int. J. Exp. Path. 82: 15-33 (2001)). Premalignant lesions are often associated with episomal forms of HPV DNA while later-stage tumors typically have integrated HPV sequences. As a result of the integration correlated with advanced stages of disease progression, the open reading frame of specific HPV genes, such as the L1 locus, may become disrupted. Such disruption of HPV gene sequence may lead to false negative results in assays designed to specifically detect the disrupted sequence.

Therefore, a preferred embodiment of the present invention provides a method for identifying the presence of a specific HPV subtype in a sample, wherein said method comprises simultaneously detecting and amplifying a plurality of HPV genes of a single HPV subtype. A sample is considered positive for the HPV subtype if a majority of the plurality of the HPV genes are detected by the methods of the present invention. Another preferred embodiment of the present invention provides an assay for the presence of a specific HPV subtype, wherein said assay comprises simultaneously detecting and amplifying three HPV genes of a single HPV subtype. A sample is considered positive for the HPV subtype if at least two of the three genes are detected and HPV negative if none of the three genes are detected by the methods of the present invention. Said assay reduces the risk of obtaining false negative results associated with assays that test for a single HPV locus. The method of the present invention is highly specific and reproducible.

The method of the present invention for detecting HPV subtypes in a clinical sample also substantially reduces the risk of false positive results as compared to other assays known in the art. Such false positive results are caused by the high degree of homology among specific HPV genes as compared to the same HPV genes from a different HPV subtype. This level of homology makes it difficult to design a PCR assay that is specific for a single HPV subtype. When utilizing other methods known in the art that detect single loci, therefore, it is necessary to confirm positive results by serially testing for the presence of several loci of a single HPV-type. The further experimentation required to verify positive results is cumbersome and time-consuming. Establishment of the HPV status of a clinical sample for four different HPV types typically consumes 26-30 man-hours.

Unlike the methods available in the art, the present invention provides a method for simultaneously detecting and amplifying a plurality of distinct HPV genes of a single HPV subtype, thus substantially reducing the occurrence of false positive results commonly associated with single-locus assays. Additionally, the assay of the present invention does not require serial experimentation to confirm positive results and greatly reduces the man-hours required to determine the HPV status of a sample. The methods of the present invention are, therefore, adaptable to high throughput screening of clinical samples for the nucleic acid of specific HPV subtypes. Said methods allow screening for numerous samples simultaneously, e.g. through use of a 96-well PCR format, but retain high specificity and accuracy.

Another HPV TaqMan assay has been described in the art that utilizes a multiple fluorophore format (Josefsson et al., Journal of Clinical Microbiology 37(3): 490-96 (1999)). This method utilizes a mixture of specific and degenerate primers to amplify a portion of the E 1 gene in a number of HPV subtypes. Up to three probes were used per assay, each probe comprising a different fluorophore and each probe detecting the E1 gene of a different HPV subtype. Assay sensitivity was tested using plasmids containing HPV DNA and not in clinical samples.

Josefsson et al. disclose a substantially reduced sensitivity in detection of HPV18 DNA when multiple fluorescent probes, each specific to a different HPV subtype, were used simultaneously as compared to a single-probe assay. Similarly, detection of HPV35 was somewhat reduced when a mixture of probes for HPV16, HPV33 and HPV35 were used, as compared to a single probe for HPV35. Additionally, somewhat reduced sensitivity was observed at high copy numbers when using a multiple probe assay to detect HPV 16 and HPV31.

The method of the present invention utilizes a plurality of fluorescent probes, each probe comprising a fluorophore that emits energy at a unique emission maxima relative to each other fluorophore used in the particular assay. The assays provided herein are highly specific and are capable of detecting fewer than ten copies of HPV genomic DNA at three loci.

The linearity and sensitivity of each PCR assay of the present invention was confirmed using loci-specific plasmids at concentrations ranging from 10 to 106 copies/reaction. The HPV6 (Fig. 6), HPV11 (Fig. 7), HPV 16 (Fig. 8) and HPV18 (Fig. 9) multiplex PCR assays were linear within the range of 10 to 10⁶ copies. The sensitivity of the HPV Multiplex PCR assays for HPV6 (Fig. 10), HPV11 (Fig. 11), HPV16 (Fig. 12), and HPV18 (Fig. 13) was also confirmed using viral DNA isolated from human clinical samples.

Tremendous assay sensitivity, as exhibited by the methods of the present invention, is critical in screening clinical samples where the copy number of HPV may be low. Because the physical manifestations of HPV infection are often covert and the latency period prolonged, infection with HPV may not be detected until the patient has been diagnosed with cervical intraepithelial neoplasia (CIN), which, if allowed to go untreated, can progress to carcinoma. Typically, higher grade lesions (CIN2, CIN3 and carcinoma) are associated with high HPV copy number, which may be detectable by traditional methods known in the art. However, many assays currently in use are not sensitive or specific enough to detect low copy number HPV. Tremendous sensitivity is critical, therefore, for early detection of HPV when HPV copy numbers are low and therapeutic intervention is more likely to be effective.

The present invention more specifically relates to a method for detecting the presence of a human papillomavirus (HPV) subtype in a nucleic acid-containing sample comprising:
amplifying the nucleic acid in the presence of a nucleic acid polymerase and a plurality of oligonucleotide sets to produce a plurality of PCR amplicons;
wherein each oligonucleotide set consists of (a) a forward discriminatory PCR primer hybridizing to a first location of an HPV subtype, (b) a reverse discriminatory PCR primer hybridizing to a second location of the HPV subtype downstream of the first location, and (c) a fluorescent probe labeled with a quencher molecule and a fluorophore which emits energy at a unique emission maxima; said probe hybridizing to a location of the HPV subtype between the first and the second locations;
wherein each oligonucleotide set specifically hybridizes to a different HPV amplicon derived from the same HPV subtype;
allowing said nucleic acid polymerase to digest each fluorescent probe during amplification to dissociate said fluorophore from said quencher molecule;
detecting a change of fluorescence upon dissociation of the fluorophore and the quencher, the change of fluorescence corresponding to the occurrence of nucleic acid amplification; and
determining that the sample is positive for the HPV subtype if a change of fluorescence is detected in at least two emission maxima.

In a preferred embodiment of this invention, each oligonucleotide set of the plurality of oligonucleotide sets is specific to a single gene of the HPV subtype to be detected. In other words, each oligonucleotide set of the method of the present invention hybridizes to nucleotide sequences derived from a single HPV gene of the same subtype. For example, the oligonucleotide primers and probe of a first oligonucleotide set hybridize to the E6 gene, the oligonucleotide primers and probe of a second oligonucleotide set hybridize to the E7 gene and the oligonucleotide primers and probe of a third oligonucleotide set hybridize to the L1 gene. As a result, a plurality of PCR amplicons is created wherein each PCR amplicon is specific to a single HPV gene of the HPV subtype to be detected.

In an alternative embodiment of this invention, the forward discriminatory PCR primer and the reverse discriminatory PCR primer of at least one oligonucleotide set are specific to a different gene of the same HPV subtype. For example, a forward discriminatory primer hybridizes to the E6 gene and a reverse discriminatory primer hybridizes to the E7 gene. As a result, at least one PCR amplicon comprises a sequence of nucleotides derived from more than one gene. The oligonucleotide probe specific to said amplicon may hybridize, for example, to a sequence of nucleotides derived from the E6 gene, a sequence of nucleotides derived from the E7 gene, or a sequence of nucleotides that crosses the E6/E7 boundary.

The change in fluorescence can be detected by an automated fluorometer designed to perform Taq-Man PCR having the following features: a method of excitation to excite the fluorophore of the fluorescent probe, a means for heating and cooling PCR reaction mixtures and a means for detecting a change in fluorescence. This combination of features, when performed by a single Taq-Man PCR instrument, allows real-time detection of PCR amplicons, which allows confirmation of PCR product amplification through examination of the kinetics of the fluorescence increase in real-time. Automated fluorometers for performing Taq-Man PCR reactions are known in the art and can be adapted for use in this specific assay, for example, the iCycler from Bio-Rad Laboratories (Hercules, CA) and the Mx4000 from Stratagene (La Jolla, CA).

The methods of the present invention were performed with an ABI PRISM® 7700 Sequence Detection Instrument (Applied Biosystems, Foster City, CA). This instrument uses a spectrograph to separate the fluorescent emission (based on wavelength) into a predictably spaced pattern across a charged-coupled device (CCD) camera. A Sequence Detection System application of the ABI PRISM^{®} 7700 collects the fluorescent signals from the CCD camera and applies data analysis algorithms.

Nucleic acid polymerases for use in the methods of the present invention must possess 5' - 3' exonuclease activity. Several suitable polymerases are known in the art, for example, Taq (*Thermus aquaticus*), Tbr (*Thermus brockianus*) and Tth (*Thermus thermophilus*) polymerases. TAQ DNA polymerase is the preferred polymerase of the present invention. The 5' - 3' exonuclease activity is characterized by the degradation of double-stranded DNA encountered during extension of the PCR primer. A fluorescent probe annealed to the amplicon will be degraded in a similar manner, thus releasing the fluorophore from the oligonucleotide. Upon dissociation of the fluorophore and the quencher, the fluorescence emitted by the fluorophore is no longer quenched, which results in a detectable change in fluorescence. During exponential growth of the PCR product, the amplicon-specific fluorescence increases to a point at which the sequence detection application, after applying a multicomponenting algorithm to the composite spectrum, can distinguish it from the background fluorescence of non-amplifying samples. The ABI PRISM® 7700 Sequence Detection Instrument also comprises a software application, which determines the threshold cycle (Ct) for the samples (cycle at which this fluorescence increases above a pre-determined threshold). PCR negative samples have a Ct equal to the total number of cycles performed and PCR positive samples have a Ct less than the total number of cycles performed.

The present invention relates to a method for detecting the presence of a human papillomavirus (HPV) subtype in a nucleic acid-containing sample. In a preferred embodiment of this invention, the HPV subtype is selected from the group consisting of: HPV6, HPV11, HPV 16 and HPV 18.

In another preferred embodiment of the method of the present invention, the number of oligonucleotide sets is odd and the sample is positive for the HPV subtype tested if a change of fluorescence is detected in a majority of fluorophores.

In yet another preferred embodiment of the method of the present invention to identify the presence of a specific HPV subtype in a sample, the number of oligonucleotide sets is three. A sample is determined to be positive for the HPV subtype if a change of fluorescence is detected in at least two fluorophores. Samples that are negative at each of the three emission maxima associated with each fluorophore are negative for the HPV subtype. This invention provides a method for detecting the presence of a human papillomavirus in a nucleic-acid containing sample where the number of oligonucleotide sets is three and the HPV subtype is selected from the group consisting of HPV6, HPV11, HPV 16 and HPV 18.

In a further preferred embodiment of the method of the present invention, the number of oligonucleotide sets is three and the oligonucleotide sets specifically hybridize to the E6, E7 and L1 genes of HPV. A sample is positive for the HPV subtype being tested if two or three of the E6, E7 or L1 genes are detected.

Oligonucleotide probes and primers of the present invention can be synthesized by a number of methods. See, e.g., Ozaki et al., Nucleic Acids Research 20: 5205-5214 (1992); Agrawal et al., Nucleic Acids Research 18: 5419-5423 (1990). For example, oligonucleotide probes can be synthesized on an automated DNA synthesizer such as the ABI 3900 DNA Synthesizer (Applied Biosystems, Foster City, CA). Alternative chemistries, e.g. resulting in non-natural backbone groups, such as phosphorothioate, phosphoramidate, and the like, may also be employed provided that the hybridization efficiencies of the resulting oligonucleotides are not adversely affected.

The PCR amplification step of the present invention can be performed by standard techniques well known in the art (See, e.g., Sambrook, E.F. Fritsch, and T. Maniatis, Molecular Cloning: A Laboratory Manual, Second Edition, Cold Spring Harbor Laboratory Press (1989); U.S. Patent No. 4,683,202; and PCR Protocols: A Guide to Methods and Applications, Innis et al., eds., Academic Press, Inc., San Diego (1990) which are hereby incorporated by reference). PCR cycling conditions typically consist of an initial denaturation step, which can be performed by heating the PCR reaction mixture to a temperature ranging from about 80°C to about 105°C for times ranging from about 1 to about 10 min. Heat denaturation is typically followed by a number of cycles, ranging from about 20 to about 50 cycles, each cycle usually comprising an initial denaturation step, followed by a primer annealing step and concluding with a primer extension step. Enzymatic extension of the primers by the nucleic acid polymerase, e.g. TAQ polymerase, produces copies of the template that can be used as templates in subsequent cycles.

"Hot start" PCR reactions may be used in conjunction with the methods of the present invention to eliminate false priming and the generation of non-specific amplicons. To this end, in a preferred embodiment of this invention, the nucleic acid polymerase is AmpliTaq Gold DNA polymerase and the PCR cycling conditions include a "hot start" PCR reaction. Said polymerase is inactive until activation, which can be accomplished by incubating the PCR reaction components at 95°C for approximately 10 minutes prior to PCR cycling. PCR methods comprising a similar initial incubation step are known in the art as "hot start" PCR assays.

Preferably, oligonucleotide probes of the present invention are in the range of about 20 to about 40 nucleotides in length. More preferably, the oligonucleotide probe is in the range of about 18 to about 30 nucleotides in length. Most preferably, the oligonucleotide probe is in the range of about 24 to about 30 nucleotides in length. The precise sequence and length of an oligonucleotide probe of the invention depends in part on the nature of the target polynucleotide to which it binds. The binding location and length may be varied to achieve appropriate annealing and melting properties for a particular embodiment.

Preferably, the 3' terminal nucleotide of the oligonucleotide probe is blocked or rendered incapable of extension by a nucleic acid polymerase. Such blocking is conveniently carried out by phosphorylation of the 3' terminal nucleotide, since the DNA polymerase can only add nucleotides to a 3' hydroxyl and not a 3' phosphate.

It is preferred that HPV primers and probes of the present invention do not share full homology with other HPV subtypes. Each primer of the present invention should be designed so that 3' homology is lacking in at least one nucleotide or more. Such primer design would substantially reduce the chance of the primer annealing to the wrong HPV subtype and prevent primer extension if annealing to an HPV type that was not intended does occur since TAQ DNA Polymerase only extends a primer from the 3' end and requires that the 3' end be properly annealed.

It is also preferred that each probe contain mismatches along the length of the oligonucleotide which destabilize the oligonucleotide binding to non-specific HPV targets. As few as one mismatch along the length of the oligonucleotide probe is enough to discriminate between loci. Because the probe of the present invention is only hydrolized and detected when bound to the segment of DNA that is being amplified, non-specific binding of the probe to a DNA sequenced that is not being amplified is not detected.

To this end, the present invention relates to a primer pair for the PCR amplification of HPV nucleic acid, wherein both the forward and reverse PCR primers are discriminatory. In a preferred embodiment of the invention, the nucleotide sequences of the primer pair are selected from the group consisting of: SEQ ID NO:1 and SEQ ID NO:2, SEQ ID NO:4 and SEQ ID NO:5, SEQ ID NO:7 and SEQ ID NO:8, SEQ ID NO:10 and SEQ ID NO:11, SEQ ID NO: 13 and SEQ ID NO: 14, SEQ ID NO: 16 and SEQ ID NO: 17, SEQ ID NO: 19 and SEQ ID NO:20, SEQ ID NO:22 and SEQ ID NO:23, SEQ ID NO:25 and SEQ ID NO:26, SEQ ID NO:28 and SEQ ID NO:29, SEQ ID NO:31 and SEQ ID NO:32, and SEQ ID NO:34 and SEQ ID NO:35.

It is readily apparent to those skilled in the art that other discriminatory oligonucleotide primers may be designed that selectively amplify HPV genes of a specific subtype. Said oligonucleotide primers may be the same length as those disclosed herein or may be in the range of 12-45 nucleotides. More preferably, the length of the oligonucleotide primers of the present invention is in the range of 18-30 nucleotides. Most preferably, the length of the oligonucleotide primers of the present invention is in the range of 19-29 nucleotides.

It is also preferred that each Taq-Man probe contain mismatches along the length of the oligonucleotide which destabilize the oligonucleotide binding to non-specific HPV targets. As few as one mismatch along the length of the oligonucleotide probe is enough to discriminate between loci. Because the probes of the present invention are only hydrolized and detected when bound to the segment of DNA that is being amplified, non-specific binding of the probe to a DNA sequenced that is not being amplified is not detected.

To this end, a preferred embodiment of this invention relates to an oligonucleotide probe comprising a sequence of nucleotides specific to a single HPV type. Said oligonucleotide probe can bind to specific HPV amplicons resulting from PCR amplification of viral DNA using specific oligonucleotide primers. In a further embodiment of this invention, said oligonucleotide probe comprises a sequence of nucleotides selected from the group consisting of: SEQ ID NO:3, SEQ ID NO:6, SEQ ID NO:9, SEQ ID NO:12, SEQ ID NO:12, SEQ ID NO:15, SEQ ID NO:18, SEQ ID NO:21, SEQ ID NO:24, SEQ ID NO:27, SEQ ID NO: 30, SEQ ID NO: 33 and SEQ ID NO:36. The present invention also relates to said oligonucleotide probes further comprising a fluorophore and a quencher molecule.

The fluorophores of the present invention may be attached to the probe at any location of the probe, including the 5' end, the 3' end or internal to either end, i.e. said fluorophore may be attached to any one of the nucleotides comprising the specific sequence of nucleotides capable of hybridizing to the specific HPV gene that the probe was designed to detect. In a preferred embodiment of this invention, the fluorophore is attached to a 5' terminal nucleotide of the specific sequence of nucleotides and the quencher is attached to a 3' terminal nucleotide of the specific sequence of nucleotides.

Preferably, fluorophores are fluorescent organic dyes derivatized for attachment to the 3' carbon or terminal 5' carbon of the probe via a linking moiety. Preferably, quencher molecules are also organic dyes, which may or may not be fluorescent, depending on the embodiment of the invention. For example, in a preferred embodiment of the invention, the quencher molecule is non-fluorescent. Generally, whether the quencher molecule is fluorescent or simply releases the transferred energy from the reporter by non-radiative decay, the absorption band of the quencher should substantially overlap the fluorescent emission band of the reporter molecule. Non-fluorescent quencher molecules that absorb energy from excited reporter molecules, but which do not release the energy radiatively, are referred to herein as "dark quenchers," "dark quencher molecules," "non-fluorescent quenchers" or "non-fluorescent quencher molecules".

Several fluorophore-quencher pairs are described in the art. See, e.g. Pesce et al, editors, Fluorescence Spectroscopy, Marcel Dekker, New York, (1971); White et al, Fluorescence Analysis: A Practical Approach, Marcel Dekker, New York, (1970); and the like. The literature also includes references providing exhaustive lists of fluorescent and non-fluorescent molecules and their relevant optical properties, e.g. Berlman, Handbook of Fluorescence Sprectra of Aromatic Molecules, 2nd Edition, Academic Press, New York, (1971). Further, there is extensive guidance in the literature for derivatizing reporter and quencher molecules for covalent attachment via common reactive groups that can be added to an oligonucleotide. See, e.g. U.S. Pat. No. 3,996, 345; and U.S. Pat. No. 4,351,760.

Exemplary fluorophore-quencher pairs may be selected from xanthene dyes, including fluoresceins, and rhodamine dyes. Many suitable forms of these compounds are widely available commercially with substituents on their phenyl moieties which can be used as the site for bonding or as the bonding functionality for attachment to an oligonucleotide. Another group of fluorescent compounds are the naphthylamines, having an amino group in the alpha or beta position. Included among such naphthylamino compounds are 1-dimethylaminonaphthyl-5-sulfonate, 1-anilino-8-naphthalene sulfonate and 2-p-touidinyl-6-naphthalene sulfonate. Other dyes include 3-phenyl-7-isocyanatocoumarin, acridines, such as 9- isothiocyanatoacridine and acridine orange; N-(p-(2- benzoxazolyl)phenyl)maleimide; benzoxadiazoles, stilbenes, pyrenes, and the like.

Preferably, fluorophore and quencher molecules are selected from fluorescein and rhodamine dyes. These dyes and appropriate linking methodologies for attachment to oligonucleotides are known in the art. See, e.g. Marshall, Histochemical J. 7:299-303 (1975); and U.S. Pat. No. 5,188,934. In a preferred embodiment of this invention, the fluorophores are selected from the group consisting of: 6-carboxy-fluorescein (FAM), 6-carboxy-4',5'-dichloro-2',7'-dimethoxyfluorescein (JOE) and 5-tetrachloro-fluorescein (TET).

In a preferred embodiment of this invention, the quencher molecule is non-fluorescent. A preferred quencher molecule of the present invention is Black Hole Quencher^{™} 1 (BHQ1), a non-fluorescent quencher developed by Biosearch Technologies (Novato, CA). In a further preferred embodiment, the fluorophores are selected from the group consisting of: FAM, JOE, and TET and the quencher molecule is BHQ1.

Preferably, commercially available linking moieties are employed that can be attached to an oligonucleotide during synthesis, e.g. available from Clontech Laboratories (Palo Alto, Calif.).

The present invention relates to a method for detecting the presence of HPV6 in a nucleic acid-containing sample comprising:
amplifying the nucleic acid in the presence of a nucleic acid polymerase and three oligonucleotide sets;
the first oligonucleotide set consisting of a forward discriminatory PCR primer as set forth in SEQ ID NO:1, a reverse discriminatory PCR primer as set forth in SEQ ID NO:2, and a probe as set forth in SEQ ID NO:3, said probe labeled with BHQ1 on the 3' end and a fluorophore on the 5' end, said fluorophore selected from the group consisting of FAM, JOE and TET;
the second oligonucleotide set consisting of a forward discriminatory PCR primer as set forth in SEQ ID NO:4, a reverse discriminatory PCR primer as set forth in SEQ ID NO:5, and a probe as set forth in SEQ ID NO:6, said probe labeled with BHQ1 on the 3' end and a fluorophore on the 5' end, said fluorophore selected from the group consisting of FAM, JOE and TET;
the third oligonucleotide set consisting of a forward discriminatory PCR primer as set forth in SEQ ID NO:7, a reverse discriminatory PCR primer as set forth in SEQ ID NO:8, and a probe as set forth in SEQ ID NO:9, said probe labeled with BHQ1 on the 3' end and a fluorophore on the 5' end, said fluorophore selected from the group consisting of FAM, JOE and TET;
allowing said nucleic acid polymerase to digest each probe during amplification to dissociate said fluorophore from said quencher molecule;
detecting a change of fluorescence upon dissociation of the fluorophore and the quencher, the change of fluorescence corresponding to the occurrence of nucleic acid amplification; and
determining that the sample is positive for the HPV6 subtype if a change of fluorescence is detected with at least two probes.

In a preferred embodiment of the method for detecting the presence of HPV6 in a sample described above, the fluorophore of the first oligonucleotide set is FAM, the fluorophore of the second oligonucleotide set is JOE and the fluorophore of the third oligonucleotide set is TET.

The present invention further relates to a method for detecting the presence of HPV11 in a nucleic acid-containing sample comprising:
amplifying the nucleic acid in the presence of a nucleic acid polymerase and three oligonucleotide sets;
the first oligonucleotide set consisting of a forward discriminatory PCR primer as set forth in SEQ ID NO:10, a reverse discriminatory PCR primer as set forth in SEQ ID NO:11, and a probe as set forth in SEQ ID NO:12, said probe labeled with BHQ1 on the 3' end and a fluorophore on the 5' end, said fluorophore selected from the group consisting of FAM, JOE and TET;
the second oligonucleotide set consisting of a forward discriminatory PCR primer as set forth in SEQ ID NO:13, a reverse discriminatory PCR primer as set forth in SEQ ID NO:14, and a probe as set forth in SEQ ID NO:15, said probe labeled with BHQ1 on the 3' end and a fluorophore on the 5' end, said fluorophore selected from the group consisting of FAM, JOE and TET;
the third oligonucleotide set consisting of a forward discriminatory PCR primer as set forth in SEQ ID NO:16, a reverse discriminatory PCR primer as set forth in SEQ ID NO:17, and a probe as set forth in SEQ ID NO:18, said probe labeled with BHQ1 on the 3' end and a fluorophore on the 5' end, said fluorophore selected from the group consisting of FAM, JOE and TET;
allowing said nucleic acid polymerase to digest each probe during amplification to dissociate said fluorophore from said quencher molecule;
detecting a change of fluorescence upon dissociation of the fluorophore and the quencher, the change of fluorescence corresponding to the occurrence of nucleic acid amplification; and
determining that the sample is positive for the HPV11 subtype if a change of fluorescence is detected with at least two probes.

In a preferred embodiment of the method for detecting the presence of HPV11 in a sample described above, the fluorophore of the first oligonucleotide set is FAM, the fluorophore of the second oligonucleotide set is JOE and the fluorophore of the third oligonucleotide set is TET.

The present invention is also related to a method for detecting the presence of HPV16 in a nucleic acid-containing sample comprising:
amplifying the nucleic acid in the presence of a nucleic acid polymerase and three oligonucleotide sets;
the first oligonucleotide set consisting of a forward discriminatory PCR primer as set forth in SEQ ID NO:19, a reverse discriminatory PCR primer as set forth in SEQ ID NO:20, and a probe as set forth in SEQ ID NO:21, said probe labeled with BHQ1 on the 3' end and a fluorophore on the 5' end, said fluorophore selected from the group consisting of FAM, JOE and TET;
the second oligonucleotide set consisting of a forward discriminatory PCR primer as set forth in SEQ ID NO:22, a reverse discriminatory PCR primer as set forth in SEQ ID NO:23, and a probe as set forth in SEQ ID NO:24, said probe labeled with BHQ1 on the 3' end and a fluorophore on the 5' end, said fluorophore selected from the group consisting of FAM, JOE and TET;
the third oligonucleotide set consisting of a forward discriminatory PCR primer as set forth in SEQ ID NO:25, a reverse discriminatory PCR primer as set forth in SEQ ID NO:26 and a probe as set forth in SEQ ID NO:27, said probe labeled with BHQ1 on the 3' end and a fluorophore on the 5' end, said fluorophore selected from the group consisting of FAM, JOE and TET;
allowing said nucleic acid polymerase to digest each probe during amplification to dissociate said fluorophore from said quencher molecule;
detecting a change of fluorescence upon dissociation of the fluorophore and the quencher, the change of fluorescence corresponding to the occurrence of nucleic acid amplification; and
determining that the sample is positive for the HPV16 subtype if a change of fluorescence is detected with at least two probes.

In a preferred embodiment of the method to detect the presence of HPV 16 in a sample described above, the fluorophore of the first oligonucleotide set is FAM, the fluorophore of the second oligonucleotide set is JOE and the fluorophore of the third oligonucleotide set TET.

This invention additionally relates to a method for detecting the presence of HPV18 in a nucleic acid-containing sample comprising:
amplifying the nucleic acid in the presence of a nucleic acid polymerase and three oligonucleotide sets;
the first oligonucleotide set consisting of a forward discriminatory PCR primer as set forth in SEQ ID NO:28, a reverse discriminatory PCR primer as set forth in SEQ ID NO:29, and a probe as set forth in SEQ ID NO:30, said probe labeled with BHQ1 on the 3' end and a fluorophore on the 5' end, said fluorophore selected from the group consisting of FAM, JOE and TET
the second oligonucleotide set consisting of a forward discriminatory PCR primer as set forth in SEQ ID NO:31, a reverse discriminatory PCR primer as set forth in SEQ ID NO:32, and a probe as set forth in SEQ ID NO:33, said probe labeled with BHQ1 on the 3' end and a fluorophore on the 5' end, said fluorophore selected from the group consisting of FAM, JOE and TET;
the third oligonucleotide set consisting of a forward discriminatory PCR primer as set forth in SEQ ID N0:34, a reverse discriminatory PCR primer as set forth in SEQ ID NO:35, and a probe as set forth in SEQ ID NO:36, said probe labeled with BHQ1 on the 3' end and a fluorophore on the 5' end, said fluorophore selected from the group consisting of FAM, JOE and TET;
allowing said nucleic acid polymerase to digest each probe during amplification to dissociate said fluorophore from said quencher molecule;
detecting a change of fluorescence upon dissociation of the fluorophore and the quencher, the change of fluorescence corresponding to the occurrence of nucleic acid amplification; and
determining that the sample is positive for the HPV 18 subtype if a change of fluorescence is detected with at least two probes.

In a preferred embodiment of the method to detect the presence of HPV18 in a sample described above, the fluorophore of the first oligonucleotide set is FAM, the fluorophore of the second oligonucleotide set is JOE and the fluorophore of the third oligonucleotide set is TET.

Having described preferred embodiments of the invention with reference to the accompanying drawings, it is to be understood that the invention is not limited to those precise embodiments, and that various changes and modifications may be effected therein by one skilled in the art without departing from the scope or spirit of the invention as defined in the appended claims.

The following examples illustrate, but do not limit the invention.

### EXAMPLE 1

### Discriminatory HPV Primer Design

PCR primers were designed for each HPV subtype using Primer Express v. 1.0 (PE Applied Biosystems, Foster City, CA). The gene-specific nucleotide sequences of the open-reading frames of the L1, E6 and E7 loci of the HPV6a, HPV6b, HPV11, HPV16, HPV18, HPV31, HPV33, and HPV45 subtypes were aligned using ClustalW v.1.7 (European Molecular Biology Laboratory, Heidelberg, Germany) and a Power Macintosh G4 personal computer (Apple Computer). The Phylip-format alignment file was then imported into the Allelic Discrimination module of the Primer Express application and the specific HPV subtype was marked.

Primer pairs were selected that met the following criteria: Tₘ = 59-61°C, amplicon size: 100-250 bp, GC content between 20-80%, a guanosine or cytosine residue at the 3'-terminal position, and the discriminatory base within the three 3'-terminal bases. The discriminatory base is the residue that is unique for the specific HPV subtype at the specific position and acts to discriminate the HPV subtype from the others in the alignment. Several primer pairs were selected such that both the sense and antisense primers were discriminatory (see FIGURE 1).

The primer sequences were analyzed for uniqueness and primer-dimer formation by *Amplify* v. 1.2 for Macintosh (William Engels, Genetics Department, University of Wisconsin). An optimal primer pair was selected for each loci in which there was no apparent dimer formation and, each primer was predicted to anneal to one and only one location of the target loci. Once an amplicon was defined by a primer pair, a dual-labeled oligonucleotide probe was designed that met the following criteria: Tₘ = 68-70°C, length ≤30 nt, runs of no more than three of the same nucleotide, no guanosine residue on the 5' terminus and more cytosine residues than guanosine residues (see FIGURE 1).

The predicted cross-reactivity of each primer and probe to other known HPV subtypes was assessed by BLAST searching each sequence against the NCBI Genbank database. Most primer and probe sequences returned unique hits for the specific HPV for which they were designed and did not share any homology with other HPV subtypes. The HPV6L1 antisense primer shares some homology with HPV3, HPV26, HPV28 and HPV70. The HPV6L1 TaqMan probe shares some homology with HPV45, HPV54, HPV59, HPV66 and HPV83. The HPV6E6 antisense primer shares homology with HPV11. The HPV6E7 antisense primer shares some homology with HPV2. The HPV6E7 TaqMan probe shares some homology with HPV11, HPV42, HPV44, HPV55 and HPV74. The HPV11L1 sense primer shares some homology with HPV6. The HPV11L1 TaqMan probe shares some homology with HPVAE8, HPV6, HPV27, HPV31, HPV34, HPV55, HPV64, and HPV71. The HPV11E6 antisense primer shares some homology with HPV57. The HPV11E6 TaqMan probe shares some homology with HPV6. The HPV11E7 sense primer shares some homology with HPV6, HPV13, HPV44, HPV55, and HPV74. The HPV11E7 TaqMan probe shares some homology with HPV6 and HPV13. The HPV16L1 sense primer shares some homology with HPV68. The HPV16L1 antisense primer shares some homology with HPV35, HPV35H, HPV42 and HPV52. the HPV16L1 TaqMan probe shares some homology with HPV7, HPV10, HPV35, and HPV35H. The HPV18E6 antisense primer shares some homology with HPV85. The HPV18E7 sense primer shares some homology with HPV6, HPV39 and HPV39. The HPV18E7 TaqMan probe shares some homology with HPV59.

None of the HPV primers and probes that were designed share full homology with other HPV subtypes. Each primer lacks 3' homology of at least one nucleotide or more which suggests that even if it were to anneal to the wrong HPV subtype, it would not be extended since TAQ DNA Polymerase only extends a primer from the 3' end and requires that the 3' end be properly annealed. Each TaqMan probe contains mismatches along the length of the oligonucleotide which destabilize the oligonucleotide binding to non-specific targets. As few as one mismatch along the length of the oligonucleotide probe is enough to discriminate between loci. In addition, the probe is only hydrolized and detected when bound to the segment of DNA that is being amplified. Non-specific binding of the probe to a DNA sequenced that is not being amplified is not detected.

### EXAMPLE 2

### Synthesis and Labeling of Oligonucleotide Primers and Probes

The oligonucleotide primers were custom synthesized and reverse-phase HPLC-purified by Sigma Genosys (The Woodlands, TX). The dual-labeled oligonucleotide probes were custom synthesized and reverse-phase HPLC-purified by Biosearch Technologies (Novato, CA). The oligonucleotide fluorescent probes for the L1 loci were 5'-labeled with 6-carboxy-fluorescein (FAM), the oligonucleotide fluorescent probes for the E6 loci were 5'-lableled with 6-carboxy-4',5'-dichloro-2',7'-dimethoxyfluorescein (JOE) and the oligonucleotide fluorescent probes for the E7 loci were 5'-lableled with 5-tetrachloro-fluorescein (TET), available from Molecular Probes (Eugene, OR). All oligonucleotide probes were 3'-labeled with BHQ1, a non-fluorescent quencher developed by Biosearch Technologies (Novato, CA). The lyophilized primers and probes were reconstituted in 1X TE pH 8.0 buffer (Roche Molecular Biochemicals) and the concentration determined by measuring the O.D. at 260 nm on a Beckman 600DU spectrophotometer and calculating the concentration using the oligonucleotide-specific molar extinction coefficient.

### EXAMPLE 3

### Optimization of the Multiplex Reaction

Primer and probe concentrations were optimized so that three separate loci could be simultaneously detected and amplified in a single PCR tube without favoring one reaction over another. The fluorescent oligonucleotide probe concentrations were optimized separately by assessing the threshold cycle (Ct) and ΔRn of increasing probe concentrations using 100 copies of DNA template (each locus cloned into a plasmid) on the ABI PRISM^{®} 7700 Sequence Detection System instrument.

Samples were amplified in a 50 µL reaction mixture containing 25 µL of the TaqMan Universal PCR 2X PCR Master Mix (Applied Biosystems, Foster City, CA), 200 nM final concentration of each primer, 100 copies of plasmid DNA template, DEPC-treated water (Ambion) and a range of concentrations (25-200 nM) of fluorescently-labeled oligonucleotide probes. The cycling conditions consisted of an initial step of 50°C for 2 min followed by 95°C for 10 min, and 45 cycles of 94°C for 15 sec and 60°C for 1 min.

Included in the Taq-Man Universal PCR master mix is dUTP (instead of dTTP) and uracil-N-glycosylase (UNG), an enzyme that is activated at 50°C and cleaves uracil-containing nucleic acids. See Longo et al., Gene 93: 125-128 (1990). UNG prevents the reamplification of carryover PCR products in subsequent experiments.

A concentration of each probe was selected that exhibited the lowest Ct (for example HPV16L1; FIGURE 2 and a ΔRn ~ 1 (for example HPV16L1; FIGURE 3). The primer concentrations were optimized for each locus by assessing the Ct and ΔRn of each primer concentration combination in a fine matrix assay using the previously determined concentration of loci-specific oligonucleotide probe and ten copies of the plasmid DNA template. The concentrations of the sense and antisense primers that exhibited the lowest Ct (for example HPV16L1; FIGURE 4) and maximal ΔRn (for example HPV16L1; FIGURE 5) were selected.

The primers and probes were then tested together with the addition of extra AmpliTaq Gold DNA Polymerase (0.75 U/well, Applied Biosystems, Foster City, CA). The additional DNA polymerase was added because the TaqMan Universal 2X PCR Master Mix, which already contains AmpliTaq Gold DNA Polymerase, was optimized for duplex reactions and not for triplex reactions. The additional DNA polymerase supplements the DNA polymerase in the 2X master mix and reinforces the reaction.

The linearity and sensitivity of each PCR assay was confirmed using loci-specific plasmids at concentrations ranging from 10 to 10⁶ copies/reaction. The HPV6 (FIGURE 6), HPV11 (FIGURE 7), HPV 16 (FIGURE 8) and HPV18 (FIGURE 9) multiplex PCR assays were linear within the range of 10 to 10⁶ copies. The sensitivity of the HPV multiplex PCR assays for HPV6 (FIGURE 10), HPV11 (FIGURE 11), HPV 16 (FIGURE 12), and HPV18 (FIGURE 13) was also confirmed using serially diluted HPV viral DNA isolated from human clinical specimens (see EXAMPLE 5).

### EXAMPLE 4

### DNA Isolation

DNA was isolated from Human clinical specimens using the QIAamp 96-well DNA Spin Blood Kit (Qiagen Inc., Valencia, CA) according to the manufacturer's protocol with the following modifications: the quantity of Qiagen protease was increased to 0.5 mg/well instead of the recommended 0.4 mg/well, the QIAamp filter plate was centrifuged dry atop a clean square-well block in a Sigma Centrifuge (Qiagen Inc, Valencia, CA) for 10 min. at 6000 RPM and the DNA was eluted with pre-warmed (70°C) elution buffer.

### EXAMPLE 5

### Screening of Human Clinical Samples

A master mix containing all of the components of the PCR reaction except the template DNA was prepared and loaded into 96-well optical reaction plates (46 µl/well, Applied Biosystems, Foster City, CA) for each HPV type being tested. Four µl of the purified DNA was added to each well containing the Multiplex PCR master mix and the wells were capped with optical PCR caps (Applied Biosystems, Foster City, CA). After centrifugation at 3000 RPM for 2 min in a Sigma centrifuge, the 96-well PCR plate was transferred to the ABI PRISM^{®} 7700 Sequence Detection Systems Instrument (Applied Biosystems, Foster City, CA).

PCR cycling and data collection were initiated and controlled by a predesigned template that is specific for each HPV subtype. When the PCR cycling was complete, the data was saved electronically and the amplification plate discarded. The data was then analyzed using the Sequence Detection Systems application (Applied Biosystems, Foster City, CA). The thresholds for each dye layer were manually set; the FAM dye layer threshold was set to 0.05, the JOE dye layer threshold was set to 0.03 and the TET dye layer was set to 0.04. The data were then exported electronically to a tab-delimited text file. The text file and the file containing the sample names was then imported into the HPV subtype-specific Microsoft EXCEL workbook via a macro which checks the data to be sure that it is of the right kind. Formulas embedded in the locked worksheets calculate dye layer PCR positivity based on the threshold cycle of each sample. Data from all three dye layers were then compiled by the workbook, which calculates a consensus HPV PCR positivity of each sample based on the rules set above.

FIGURES 10-13 show the sensitivity of the HPV multiplex PCR assays for HPV6, HPV11, HPV16, and HPV18, respectively, using serially diluted HPV viral DNA isolated from human clinical specimens. High concentration viral DNA from clinical samples was diluted 10-fold serially in a background of human genomic DNA and run in the appropriate multiplex PCR assay. After a 100,000 to 1,000,000-fold dilution (approximate copy number 1-10), viral DNA was still detectable. Viral detection at low copy numbers was linear over the entire dilution range.

## Claims

1. An oligonucleotide probe comprising a sequence of nucleotides selected from the group consisting of: SEQ ID NO:3, SEQ ID NO:6, SEQ ID NO:9, SEQ ID NO:12, SEQ ID NO:12, SEQ ID NO:15, SEQ ID NO:18, SEQ ID NO:21, SEQ ID NO:24, SEQ ID NO:27, SEQ ID NO: 30, SEQ ID NO: 33 and SEQ ID NO:36.

2. The oligonucleotide probe of claim 1 further comprising a fluorophore and a quencher molecule.

3. The oligonucleotide probe of claim 2, wherein the fluorophore is attached to a 5' terminal nucleotide of the sequence of nucleotides and the quencher is attached to a 3' terminal nucleotide of the sequence of nucleotides.

4. The oligonucleotide probe of claim 2, wherein the fluorophore is selected from the group consisting of: FAM, JOE and TET.

5. The oligonucleotide probe of claim 2, wherein the quencher molecule is non-fluorescent.

6. The oligonucleotide probe of claim 3, wherein the quencher molecule is BHQ1.

7. A primer pair for the PCR amplification of HPV nucleic acid, wherein the nucleotide sequences of the primer pair are selected from the group consisting of:
SEQ ID NO:1 and SEQ ID NO:2,
SEQ ID NO:4 and SEQ ID NO:5,
SEQ ID NO:7 and SEQ ID NO:8,
SEQ ID NO:10 and SEQ ID NO:11,
SEQ ID NO:13 and SEQ ID NO:14,
SEQ ID NO:16 and SEQ ID NO:17,
SEQ ID NO:19 and SEQ ID NO:20,
SEQ ID NO:22 and SEQ ID NO:23,
SEQ ID NO:25 and SEQ ID NO:26,
SEQ ID NO:28 and SEQ ID NO:29,
SEQ ID NO:31 and SEQ ID NO:32, and
SEQ ID NO:34 and SEQ ID NO:35.
